# EUROPÄISCHE PATENTSCHRIFT

(11) **EP 2 480 522 B1**
(45) Veröffentlichungstag und Bekanntmachung des Hinweises auf die Patenterteilung: **26.02.2014**
(21) Anmeldenummer: 10754699.6
(22) Anmeldetag: 18.09.2010
(51) Int. Cl.: C07C 225/22, C07C 317/36, A01N 35/06, A01N 41/10

(54) **3-AMINO-2-NITRO-SUBSTITUIERTE BENZOYLDERIVATE UND IHRE VERWENDUNG ALS HERBIZIDE**
3-AMINO-2-NITRO SUBSTITUTED BENZOYL DERIVATIVES AND USE OF SAME AS HERBICIDES
DÉRIVÉS DE BENZOYLE 3-AMINO-2-NITRO SUBSTITUÉS ET LEUR UTILISATION COMME HERBICIDES

(30) Priorität: 25.09.2009 EP 09012170; 28.09.2009 US 246305 P
(43) Veröffentlichungstag der Anmeldung: 01.08.2012
(73) Patentinhaber: Bayer CropScience AG, 40789 Monheim (DE)
(72) Erfinder: VAN ALMSICK, Andreas, 61184 Karben (DE); DITTGEN, Jan, 60316 Frankfurt (DE); ROSINGER, Christopher, Hugh, 65719 Hofheim (DE); HÄUSER-HAHN, Isolde, 51375 Leverkusen (DE); FEUCHT, Dieter, 65760 Eschborn (DE)
(86) Internationale Anmeldenummer: PCT/EP2010/005740
(87) Internationale Veröffentlichungsnummer: WO 2011/035875

(56) Entgegenhaltungen:
- EP-A1- 1 188 376
- WO-A1-2005/123667
- DE-A1- 19 935 218
- US-A- 5 006 158
- US-A- 5 801 120
- BARTA, I.C. ET AL.: "BENZOYLCYCLOHEXANEDIONE HERBICIDES ARE STRONG INHIBITORS OF PURIFIED P-HYDROXYPHENYLPYRUVIC ACID DIOXYGENASE OF MAIZE" PESTICIDE SCIENCE, Bd. 45, Nr. 3, 1. November 1995 (1995-11-01), Seiten 286-287, XP000547268 ISSN: 0031-613X

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte 2-Nitro-substituierte Benzoylderivate herbizide Eigenschaften besitzen. So werden in US 4,780,127, US 5,006,158, EP 186 118 A1 und EP 338 992 A1 2-Nitro-substituierte Benzoylderivate beschrieben, die am Phenylring durch weitere Reste substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung von weiteren herbizid wirksamen Verbindungen mit - gegenüber den aus dem Stand der Technik bekannten Verbindungen - verbesserten Eigenschaften.

Es wurde nun gefunden, daß 3-Amino-2-nitro-substituierte Benzoylderivate als Herbizide besonders gut geeignet sind.

Ein Gegenstand der vorliegenden Erfindung sind 3-Amino-2-nitro-substituierte Benzoylderivate der Formel (I) oder deren Salze 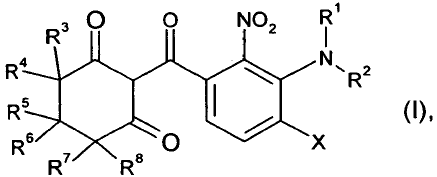 worin
X bedeutet Halogen, Ethylsulfonyl, Methylsulfonyl, 1,2,4-Triazol-1-yl oder 1,2,4-Triazol-4-yl,
R¹ und R² bedeuten unabhängig voneinander jeweils Wasserstoff, (C₁-C₆)-Alkyl, (C₂-C₆)-Alkenyl, (C₂-C₆)-Alkinyl, Halogen-(C₁-C₆)-alkyl, Halogen-(C₁-C₆)-alkenyl, Halogen-(C₁-C₆)-alkinyl, (C₃-C₆)-Cycloalkyl-(C₁-C₆)-alkyl, (C₃-C₆)-Cycloalkenyl-(C₁-C₆)-alkyl, (C₁-C₆)-Alkoxy-(C₁-C₆)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂ oder CH=CH,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder lod.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Bevorzugt sind Verbindungen der allgemeinen Formel (I), worin
X bedeutet Chlor, Brom, Fluor oder Methylsulfonyl,
R¹ und R² bedeuten unabhängig voneinander jeweils Wasserstoff, (C₁-C₆)-Alkyl, Halogen-(C₁-C₆)-alkyl, Halogen-(C₁-C₆)-Alkenyl, (C₃-C₆)-Cycloalkyl-(C₁-C₆)-alkyl oder (C₁-C₆)-Alkoxy-(C₁-C₆)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

Besonders bevorzugt sind Verbindungen der allgemeinen Formel (I), worin
X bedeutet Chlor oder Brom,
R¹ bedeutet Wasserstoff oder Methyl,
R² bedeutet Wasserstoff, (C₁-C₄)-Alkyl, Halogen-(C₁-C₄)-alkyl, (C₃-C₆)-Cycloalkyl-(C₁-C₄)-alkyl oder (C₁-C₄)-Alkoxy-(C₁-C₄)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

Die erfindungsgemäßen Verbindungen der Formel (I) können in Abhängigkeit von äußeren Bedingungen, wie Lösungsmittel und pH-Wert, in unterschiedlichen tautomeren Strukturen auftreten:

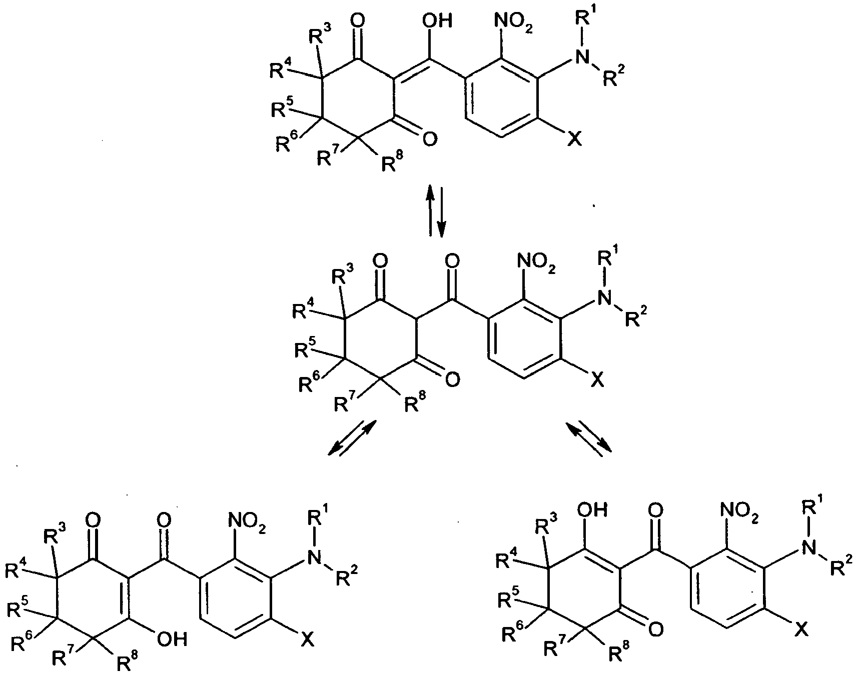

In allen nachfolgend genannten Formeln haben die Substituenten und Symbole, sofern nicht anders definiert, dieselbe Bedeutung wie unter Formel (I) beschrieben.

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Ebenso können Salze gebildet werden durch Anlagerung von organischen Säuren, wie Ameisen- oder Essigsäure, und anorganischen Säuren, wie Phosphor-, Salz- oder Schwefelsäure. Solche Salze sind ebenfalls Gegenstand der Erfindung.

Erfindungsgemäße Verbindungen der Formel (I) erhält man beispielsweise durch basenkatalysierte Reaktion von 1,3-Diketone der Formel (II) mit Benzoesäurederivaten der Formel (III), worin L für Halogen, Hydroxy oder Alkoxy steht, und anschließender Cyanid induzierter Umlagerung. Wenn L für Hydroxy steht, wird vorzugsweise in Anwesenheit eines Wasser entziehenden Mittels, wie Dicyclocarbodümid, gearbeitet. Als Cyanid-Quelle für die Umlagerung der Enolester (III) zu den erfindungsgemäßen Verbindungen (I) dient beispielsweise Kaliumcyanid, Acetoncyanhydrin und Trimethylsilylcyanid, vorzugsweise in einer Menge von 1 bis 50 Molprozent. Beispiele zur cyanidkatalysierten Umlagerung von Enolestern findet man z.B. in WO 00/21924 oder US 6,297,196.

Die als Ausgangsmaterial verwendeten 1,3-Diketone der Formel (II) sind bekannt, kommerziell erhältlich oder können nach an sich bekannten Verfahren hergestellt werden, wie sie beispielsweise in der EP-A 71707, EP-A 142741, EP-A 243313, US 4,249,937, WO 92/13821, WO 2002/006197 und WO 2005/123667 beschrieben sind.

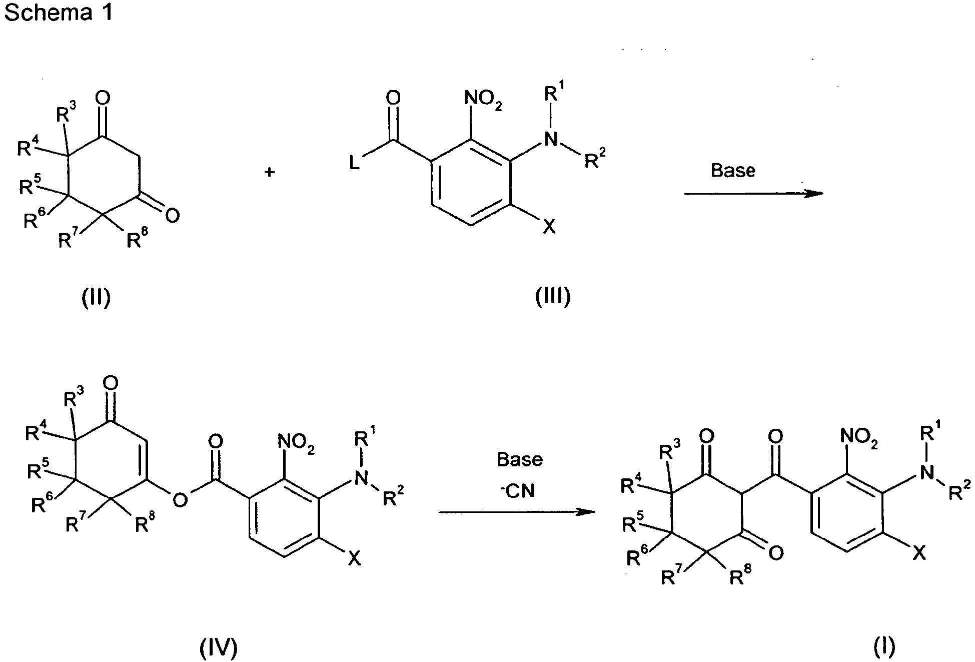

Die Benzoesäurederivate (III) können beispielsweise gemäß Schemata 2 bis 4 durch dem Fachmann grundsätzlich bekannte Reaktionen aus den Verbindungen (V) hergestellt werden. Danach lassen sich z.B. wie in Schema 2 dargestellt 3-Aminobenzoesäuren (V), worin mit X für Halogen steht, in an sich bekannter Weise durch Umsetzung z.B. mit Essigsäureanhydrid und einer katalytischen Menge konz. Schwefelsäure in die entsprechenden Acetamide (VI) überführen. Die Überführung in den entsprechenden Estern (VII), worin L für Alkoxy steht, gelingt mit den üblichen bekannten Veresterungsmethoden, vorteilhafterweise aber mit Umsetzung von Dimethyl- oder Diethylsulfat in Gegenwart von Kaliumcarbonat in einem geeigneten Lösungsmittel wie N,N-Dimethylformamid. Die anschließende Nitrierung führt zu Verbindungen (VIII), die mit einem Schwefelsäure/Alkohol Gemisch zu den entsprechenden 3-Amino-2(6)-Nitro-benzoesäureestern (IX) umgesetzt werden.

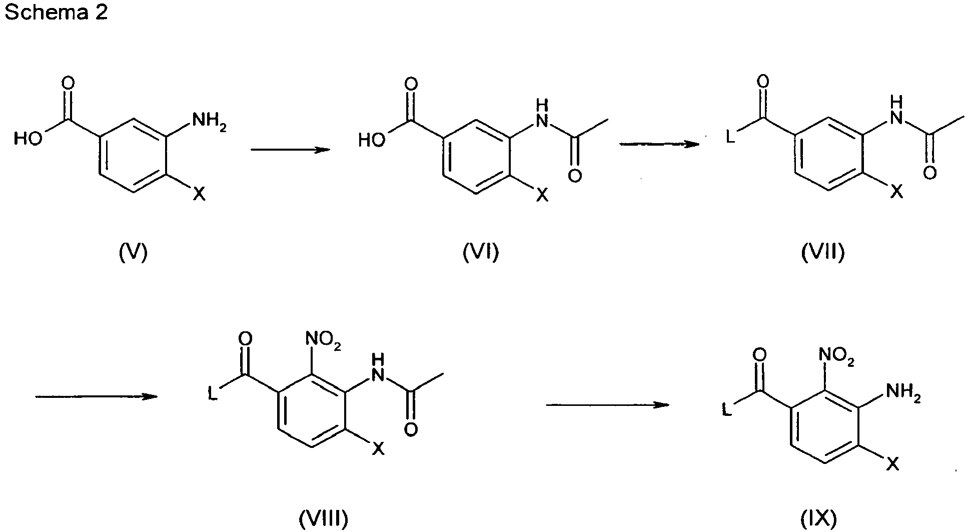

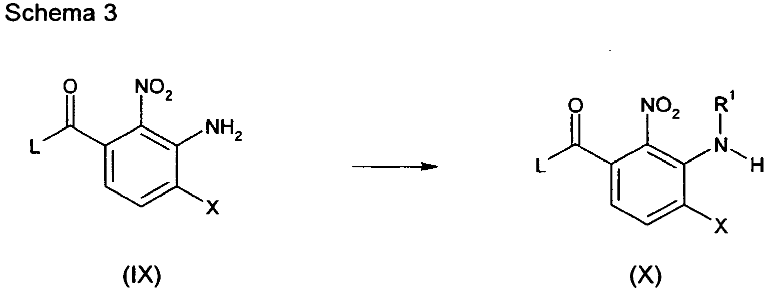

Gemäß Schema 3 wird ein 3-Amino-2-Nitro-benzoesäureester der allgemeinen Formel (IX) mit einem geeigneten Methylierungsmittel wie Methyliodid in Gegenwart einer geeigneten starken Base wie Natriumhydrid in einem geeigneten Lösungsmittel wie DMF zum Monomethylaminderivat (X) umgesetzt.

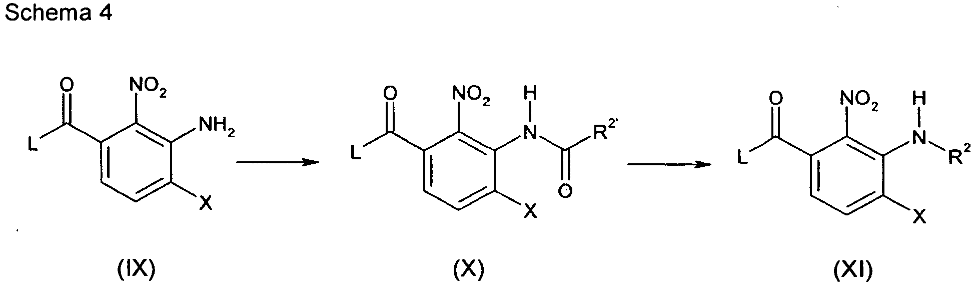

Gemäß Schema 4 werden 3-Amino-2-Nitro-benzoesäureester der allgemeinen Formel (IX) an sich bekannten Methoden mit geeigneten Acylierungsmitteln, wie Anhydriden oder Säürechloriden, unter geeigneten Bedingungen in die entsprechenden Amide (X) überführt. Darin steht R^{2'} für den um eine CH₂-Einheit verkürzten Rest R². Die Verbindungen (XI) können dann nach dem in Schema 3 genannten Verfahren durch Einführen des Rests R¹ zu den Verbindungen (III) umgesetzt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chrornatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipornoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird. Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuronmethyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), lodosulfuron, lodosulfuron-methyl-natrium, loxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, - ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuronmethyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

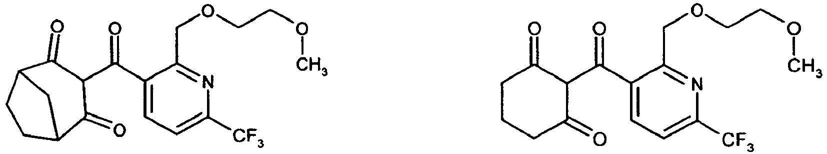

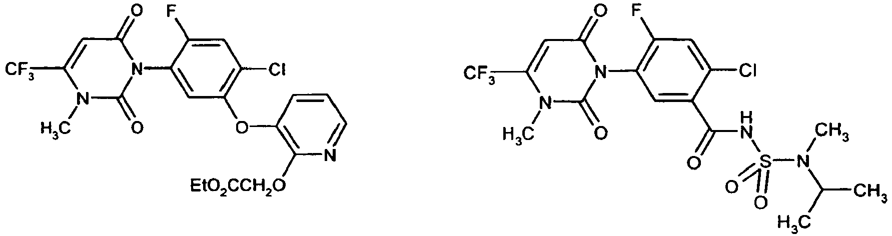

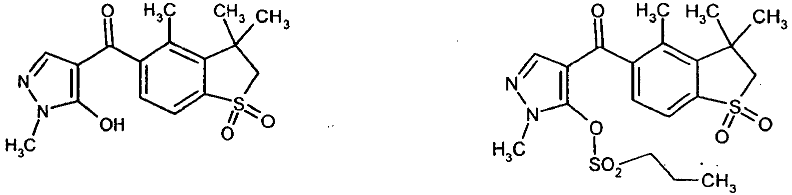

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 2-[4-Chlor-3-(methylamino)-2-nitrobenzoyl]-5-methylcyclohexan-1,3-dion, (Tabellenbeispiel Nr. 2-2)

### Schritt 1: Synthese von 3-Acetamido-4-chlorbenzoesäure

### Tetrahydrofuran (THF Raumtemperatur (RT), NaHCO₃-Lösung

101.9 g (582 mmol) 3-Amino-4-chlorbenzoesäure wurden in 700 ml Essigsäure suspendiert und anschließend mit 64.8 ml (687 mmol) Essigsäureanhydrid versetzt. Das Gemisch wurde 15 h unter Rückfluss erhitzt und nach dem Abkühlen auf 1,5 l Eiswasser gegeben. Die ausgefallenen Kristalle wurden abgesaugt, mit Wasser nachgewaschen und im Vakuumschrank 17 h bei 70 °C getrocknet.
¹H-NMR (DMSO): δ 2.12 (s, 3H), 7.6 (d, 1 H), 7.7 (dd, 1 H), 8.31 (s, 1 H), 9.63 (s, 1H), 13.18 (s, 1 H)
Ausbeute: 120.09 g (562 mmol), 97 % hellbeige Kristalle

### Schritt 2: Synthese von Ethyl-3-acetamido-4-chlorbenzoat

120 g (562 mmol) 3-Acetamido-4-chlorbenzoesäure wurden in 77 ml DMF gelöst und mit 93.16 g (674 mmol) Kaliumcarbonat 30 min bei 60 °C gerührt. Anschließend ließ man abkühlen, gab 91 g (590 mmol) Diethylsulfat hinzu und ließ 7 h bei RT rühren. Der Ansatz wurde eingeengt, der Rückstand mit 2 l Wasser verrührt und die ausgefallenen Kristalle abgesaugt. Es wurde mit Wasser nachgewaschen und 3 h bei 70 °C im Vakuumschrank getrocknet.
¹H-NMR (CDCl3): δ 1.4 (t, 3H), 2.26 (s, 3H), 4.38 (q, 2H), 7.44 (d, 1H), 7.6 (s, br, 1 H), 7.75 (dd, 1 H), 8.95 (s, br, 1 H)
Ausbeute: 133.40 g (552 mmol) 97 % hellbeige Kristalle

### Schritt 3: Synthese von Ethyl-3-acetamido-4-chlor-2-nitrobenzoat

133,4 g (552 mmol) Ethyl-3-acetamido-4-chlorbenzoat wurden bei -10 °C vorgelegt und unter Kühlung mit 207 ml 100 % iger Salpetersäure zwischen -10 bis 0 °C versetzt. Es wurde 2 h bei -5 °C nachgerührt. Danach wurde auf 2.1 l Eiswasser gegeben, die ausgefallenen Kristalle abgesaugt, mit Wasser nachgewaschen und bei 60 °C im Vakuumschrank getrocknet.
Ausbeute: 138.63 g hellbraune Kristalle, bestehend aus einem Gemisch aus 40.2 % Ethyl-3-acetamido-4-chlor-2-nitrobenzoat, 35.9 % Ethyl-5-acetamido-4-chlor-2-nitrobenzoat und 13.6 % Edukt

### Schritt 4: Synthese von Ethyl-3-amino-4-chlor-2-nitrobenzoat

138.63 g des Gemisches aus Schritt 3 wurden in 1.4 l Ethanol und 60 ml konz. Schwefelsäure gelöst und 25 h bei Rückfluss gekocht. Danach wurden 1.3 l Ethanol am Rotationsverdampfer abrotiert, der Rückstand mit 1 l Eiswasser versetzt, mit gesättigten NaHCO₃ Lösung basisch gestellt und anschließend dreimal mit jeweils 500 ml Dichlormethan extrahiert. Die vereinigten organischen Phasen wurden über MgSO₄ getrocknet, über Kieselgel abgesaugt und eingeengt. Anschließend wurde chromatographisch getrennt (Kieselgel, Heptan/Essigsäureethylester 9:1).
¹H-NMR (CDCl3): δ 1.35 (t, 3H), 4.35 (q, 2H), 6 (s, br, 2H), 6.88 (d, 1 H), 7.5 (d, 1 H) Ausbeute: 53.0 g (217 mmol) orangenes Öl

### Schritt 5: Synthese von Ethyl-4-chlor-3-(methylamino)-2-nitrobenzoat

3.18 g (13.0 mmol) Ethyl-3-amino-4-chlor-2-nitrobenzoat wurden in DMF vorgelegt und unter einer Stickstoffatmosphäre mit 0.624 g (15.6 mmol) Natriumhydrid versetzt. Man ließ 30 min rühren und gab dann 2.21 g (15.6 mmol) Methyliodid hinzu. Nach weiteren 2.5 h Rühren bei Raumtemperatur wurde eingeengt, der Rückstand mit Essigsäureethylester extrahiert, mit Wasser gewaschen und anschließend über MgSO₄ getrocknet. Anschließend wurde chromatographisch getrennt (Kieselgel, Heptan/Essigsäureethylester 9:1).
¹H-NMR (CDCl3): δ 1.33 (t, 3H), 2.91 (d, 1 H), 4.33 (q, 2H), 4.8 (s, br, 1 H), 7.2 (d, 1 H), 7.42 (d, 1 H)
Ausbeute: 3.01 g (11.6 mmol) 86 % orangenes Öl

### Schritt 6: Synthese von 4-Chlor-3-(methylamino)-2-nitrobenzoesäure

3.01 g (11.6 mmol) Ethyl-4-chlor-3-(methylamino)-2-nitrobenzoat wurden in 35 ml Tetrahydrofuran und 22 ml Wasser gelöst und mit 0.5 g (20.9 mmol) Lithiumhydroxid versetzt. Man ließ 8 h rühren und engte anschließend ein. Der Rückstand wurde in Wasser aufgenommen, mit Methylenchlorid gewaschen und die wässrige Phase anschließend mit einer KHSO₄ Lösung auf pH 1 eingestellt. Der Niederschlag wurde abgesaugt, mit Wasser gewaschen und im Vakuum bei 70 °C getrocknet.
¹H-NMR (CDCl3): δ 2.91 (s, 3H), 7.32 (d, 1H), 7.45 (d, 1 H)
Ausbeute: 2.30 g (10.0 mmol) 86 % orangene Kristalle

### Schritt 7: Synthese von 5-Methyl-3-oxocyclohex-1-en-1-yl-3-amino-4-chlor-2-nitrobenzoat

250 mg (1.1 mmol) 4-Chlor-3-(methylamino)-2-nitrobenzoesäure, 410.3 mg (3.3 mmol) 5-Methyl-cyclohexan-1,3-dion, 275.7 mg (1.4 mmol) N-(3-Dimethylaminopropyl)-N'-ethylcarbodiimid-hydrochlorid und 13.5 mg (0.1 mmol) 4-(Dimethylamino)-pyridin wurden in 6 ml Methylendichlorid gelöst und 8 h bei RT gerührt. Anschließend wurde mit Wasser und ges. NaHCO₃-Lösung gewaschen, die org. Phase über MgSO₄ getrocknet und danach alles über eine kleine Glasfritte, mit 1 cm Kieselgel gefüllt abgesaugt und mit Heptan/Essigsäureethylester 1:2 nachgewaschen. Das Filtrat wurde eingeengt.
¹H-NMR (CDCl3): δ 1.15 (d, 3H), 2.15 (m, 1 H), 2.3-2.6 (m, 4H), 2.96 (d, 3H), 5.08 (d, br, 1 H), 6.0 (d, 1 H), 7.2 (d, 1 H), 7.49 (d, 1 H)
Ausbeute: 296.5 mg (0.88 mmol) 81 %

### Schritt 8: Synthese von 2-[4-Chlor-3-(methylamino)-2-nitrobenzoyl]-5-methylcyclohexan-1,3-dion

290 mg (0.86 mmol) 5-Methyl-3-oxocyclohex-1-en-1-yl-3-amino-4-chlor-2-nitrobenzoat, 164.6 mg (1.6 mmol) Triethylamin, 23.4 mg (0.36 mmol) Kaliumcyanid und 7.3 mg (0.086 mmol) Acetoncyanhydrin wurden in 15 ml Acetonitril 24 h bei RT gerührt. Anschließend wurde eingeengt, der Rückstand mit 30 ml ges. KHSO₄-Lösung versetzt und mit Methylenchlorid extrahiert. Die vereinigten organischen Phasen wurden über MgSO₄ getrocknet und über eine kleine Glasfritte mit 5 cm Kieselgel gefüllt abfiltriert und mit Essigsäureethylester nachgewaschen. Das Filtrat wurde eingeengt.
¹H-NMR (CDCl3): δ 1.1 (d, 3H), 2.08-2.18 (m, 1 H), 2.22-2.35 (m, 1H), 2.4-2.51 (m, 1 H), 2.75-2.82 (m, 1 H), 3.06 (d, 3H), 6.45 (d, 1 H), 6.52 (s, br, 1 H), 7.45 (d, 1 H), 16.45 (s, 1 H)
Ausbeute: 98 mg (0.29 mmol) 34 %

### Herstellung von 2-{4-Brom-3-[(2,2-dimethylpropyl)amino]-2-nitrobenzoyl}cyclohexan-1,3-dion, (Tabellenbeispiel Nr. 1-23)

### Schritt 1: Synthese von Methyl-4-brom-3-[(2,2-dimethylpropanoyl)amino]-2-nitrobenzoat

3.0 g (10.9 mmol) Methyl-3-amino-4-brom-2-nitrobenzoat wurden mit 5 ml Pivalinsäureanhydrid vermischt und unter Rückfluss mit 0.06 ml konz. Schwefelsäure versetzt. Man ließ eine Stunde kochen und anschließend abkühlen. Der Ansatz wurde mit Wasser verdünnt und der ausgefallene Feststoff abgesaugt. Anschließend wurde dieser mit Wasser und Heptan gewaschen und über Kieselgel mit Methylenchlorid gelfiltriert.
¹H-NMR (CDCl₃): δ 1.31 (s, 9H), 3.9 (s, 3H), 7.3 (s, br, 1H), 7.75 (d, 1H), 7.85 (d, 1 H)
Ausbeute: 3.81 g (10.6 mmol) 97 % gelbe Kristalle

### Schritt 2: Synthese von Methyl-4-brom-3-[(2,2-dimethylpropyl)amino]-2-nitrobenzoat

3.76 g (10.47 mmol) Methyl-4-brom-3-[(2,2-dimethylpropanoyl)amino]-2-nitrobenzoat wurden in Toluol suspendiert und mit 6 ml (11.26 mmol) Boran-Dimethylsulfid (2M in Toluol) versetzt. Man ließ 15 h unter Rückfluss rühren und anschließend abkühlen. Danach wurden 50 ml ges. NaHCO₃-Lösung zugesetzt, nochmals 30 min bei RT gerührt und anschließend die organische Phase abgetrennt, über MgSO₄ getrocknet und eingeengt. Anschließend wurde chromatographisch getrennt (Kieselgel, Heptan/Essigsäureethylester 4:1).
¹H-NMR (CDCl₃): ö 0.98 (s, 9H), 2.85 (d, 2H), 3.89 (s, 3H), 4.58 (s, br, 1 H), 7.15 (d, 1 H), 7.62 (d, 1 H)
Ausbeute: 0.89 g (2.58 mmol) 25 % gelbe Kristalle

### Schritt 3: Synthese von 4-Brom-3-[(2,2-dimethylpropyl)amino]-2-nitrobenzoesäure

0.88 g (2.55 mmol) Methyl-4-brom-3-[(2,2-dimethylpropyl)amino]-2-nitrobenzoat wurden in 10 ml Tetrahydrofuran und 10 ml Wasser gelöst und mit 0.064 g (2.67 mmol) Lithiumhydroxid versetzt. Man ließ 15 h rühren und engte anschließend ein. Der Rückstand wurde in Wasser aufgenommen, mit Methylenchlorid gewaschen und die wässrige Phase mit einer KHSO₄ Lösung auf pH 1 eingestellt. Der Niederschlag wurde abgesaugt, mit Wasser gewaschen und im Vakuum bei 70 °C getrocknet. ¹H-NMR (DMSO): δ 2.50 (s, 9H), 2.75 (d, 2H), 4.68 (t, br, 1H), 7.23 (d, 1 H), 7.88 (d, 1 H), 13.85 (s, br, 1 H)
Ausbeute: 0.812 g (2.45 mmol) 96 % gelbe Kristalle

### Schritt 4: Synthese von 3-Oxocyclohex-1-en-1-yl-4-brom-3-[(2,2-dimethylpropyl)amino]-2-nitrobenzoat

190 mg (0.574 mmol) 4-Brom-3-[(2,2-dimethylpropyl)amino]-2-nitrobenzoesäure, 198 mg (1.713 mmol) Cyclohexan-1,3-dion, 145 mg (0.741 mmol) N-(3-Dimethylaminopropyl)-N'-ethylcarbodiimid-hydrochlorid und 7 mg (0.057 mmol) 4-(Dimethylamino)-pyridin wurden in 5 ml Methylendichlorid gelöst und 24 h bei RT gerührt. Anschließend wurde mit Wasser und ges. NaHCO₃-Lösung gewaschen, die org. Phase über MgS04 getrocknet und danach alles über eine kleine Glasfritte, mit 1 cm Kieselgel gefüllt abgesaugt und mit Heptan/Essigsäureethylester 1:2 nachgewaschen. Das Filtrat wurde eingeengt.
¹H-NMR (CDCl₃): δ 0.98 (s, 9H), 2.10 (m, 2H), 2.45 (m, 2H), 2.62 (m, 2H), 2.94 (d, 2H), 5.01 (t, br, 1 H), 6.0 (s, 1 H), 7.20 (d, 1 H), 7.50 (d, 1 H)
Ausbeute: 68 mg (0.16 mmol) 28 % gelbes Öl

### Schritt 5: Synthese von 2-{4-Brom-3-[(2,2-dimethylpropyl)amino]-2-nitrobenzoyl}-cyclohexan-1,3-dion

68 mg (0.144 mmol) 3-Oxocyclohex-1-en-1-yl-4-brom-3-[(2,2-dimethylpropyl)amino]-2-nitrobenzoat, 29 mg (0.287 mmol) Triethylamin, 4 mg (0.061 mmol) Kaliumcyanid und 1 mg (0.015 mmol) Acetoncyanhydrin wurden in 3 ml Acetonitril 60 h bei Raumtemperatur gerührt. Anschließend wurde eingeengt, der Rückstand mit 30 ml ges. KHSO₄-Lösung versetzt und mit Methylenchlorid extrahiert. Die vereinigten organischen Phasen wurden über MgSO₄ getrocknet und über eine kleine Glasfritte mit 5 cm Kieselgel gefüllt abfiltriert und mit Heptan/Essigsäureethylester 1:2 nachgewaschen. Das Filtrat wurde eingeengt.
¹H-NMR (CDCl₃): δ 0.98 (s, 9H), 2.03 (m, 2H), 2.39 (m, 2H), 2.78 (m, 2H), 3.12 (d, 2H), 6.42 (d, 1 H), 7.45 (d, 1 H), 16.50 (s, 1 H)
Ausbeute: 56 mg (0.118 mmol) 82 % gelbes Öl

Die in nachfolgenden Tabellen aufgeführten Beispiele wurden analog oben genannten Methoden hergestellt beziehungsweise sind analog oben genannten Methoden erhältlich. Diese Verbindungen sind jeweils ganz besonders bevorzugt.

Die verwendeten Abkürzungen bedeuten:
Et = Ethyl Me = Methyl Pr = Propyl Bu = Butyl

**Tabelle 1: Erfindungsgemäße Verbindungen der allgemeinen Formel (I), worin R³, R⁴, R⁵, R⁶, R⁷und R⁸ jeweils für Wasserstoff stehen**

| | | | | |
|---|---|---|---|---|
| 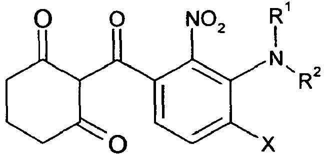 | | | | |

| Nr. | X | R¹ | R² | Physikalische Daten: ¹H-NMR: δ [CDCl₃] |
|---|---|---|---|---|
| 1-1 | Cl | H | H | 2.02 (m, 2H), 2.38 (t, 2H), 2.78 (t, 2H), 6.38 (d, 1H), 6.65 (s, br, 2H), 7.51 (d, 1H), 16.45 (s, 1H) |
| 1-2 | Cl | H | Me | 2.03 (m, 2H), 2.4 (t, 2H), 2.78 (t, 2H), 3.08 (s, 3H), 6.43 (d, 1H), 6.52 (s, br, 1H), 7.45 (d, 1H), 16.48 (s, 1H) |
| 1-3 | Cl | Me | Me | 2.05 (m, 2H), 2.42 (t, 2H), 2.78 (t, 2H), 2.85 (s, 6H), 6.88 (d, 1H), 7.5 (d, 1H), 16.45 (s, 1H) |
| 1-4 | Cl | H | Et | 1.25 (t, 3H), 2.05 (m, 2H), 2.4 (t, 2H), 2.78 (t, 2H), 3.4 (q, 2H), 6.48 (d, 1H), 7.46 (d, 1H), 16.5 (s, 1H) |
| 1-5 | Cl | H | nPr | 0.95 (t, 3H), 1.65 (q, 2H), 2.02 (m, 2H), 2.40 (t, 2H), 2.75 (t, 2H), 3.32 (t, 2H), 6.45 (d, 1H), 7.45 (d, 1H), 16.5 (s, br, 1H) |
| 1-6 | Cl | H | nBu | 0.92 (t, 3H), 1.39 (m, 2H), 1.6 (m, 2H), 2.05 (m, 2H), 2.39 (m, 2H), 2.75 (m, 2H), 3.36 (t, 2H), 3.2 (t, 2H), 6.45 (d, 1H), 6.48 (s, br, 1H), 7.45 (d, 1H), 16.48 (s, 1H) |
| 1-7 | Cl | H | CH₂iPr | 0.95 (d, 6H), 1.85 (m, 1H), 2.02 (m, 2H), 2.4 (t, 2H), 2.76 (7, 2H), 3.19 (m, 2H), 6.45 (d, 2H), 7.45 (d, 2H), 16.48 (s, 1H) |
| 1-8 | Cl | H | CH₂cPr | 0.25 (m, 2H), 0.55 (m, 2H), 1.05 (m, 1H), 2.03 (m, 2H), 2.39 (m, 2H), 2.78 (m, 2H), 3.18 (d, 2H), 6.48 (d, 1H), 6.55 (s, br, 1H), 7.45 (d, 1H), 16.50 (s, 1H) |
| 1-9 | Cl | H | CH₂tBu | 0.98 (s, 9H), 2.03 (m, 2H), 2.39 (m, 2H), 2.78 (m, 2H), 3.12 (d, 2H), 6.45 (d, 1H), 7.45 (d, 1H), 16.50 (s, 1H) |
| 1-10 | Cl | H | CH₂CH₂iPr | 0.90 (d, 6H), 1.5 (m, 2H), 1.68 (m, 1H), 2.05 (m, 2H), 2.4 (m, 2H), 2.75 (m, 2H), 3.38 (m, 2H), 6.45 (s, br, 1H), 6.45 (d, 1H), 7.45 (d, 1H), 16.48 (s, 1H) |
| 1-11 | Cl | H | CH₂CH₂OMe | |
| 1-12 | Cl | H | CH₂CH₂OEt | |
| 1-13 | Cl | H | CH₂CH₂CH₂OMe | |
| 1-14 | Cl | H | CH₂CH₂CH₂OMe | |
| 1-15 | Br | H | H | 2.02 (m, 2H), 2.38 (t, 2H), 2.78 (t, 2H), 6.3 (d, 1H), 6.7 (s, br, 2H), 7.68 (d, 1H), 16.45 (s, br, 1H) |
| 1-16 | Br | H | Me | 2.02 (m, 2H), 2.4 (t, 2H), 2.78 (t, 2H), 2.98 (s, 3H), 6.4 (d, 1H), 7.62 (d, 1H), 16.48 (s, 1H) |
| 1-17 | Br | Me | Me | δ [MeOD] = 2.05 (m, 2H), 2.62 (m, 4H), 2.85 (s, 6H), 7.01 (d, 1H), 7.8 (d, 1H) |
| 1-18 | Br | H | Et | 1.28 (t, 3H), 2.02 (m, 2H), 2.4 (t, 2H), 2.75 (t, 2H), 3.28 (q, 2H), 5.8 (s, br, 1H), 6.42 (d, 1H), 7.65 (d, 1H), 16.48 (s, 1H) |
| 1-19 | Br | H | nPr | 0.98 (t, 3H), 1.62 (m, 2H), 2.02 (m, 2H), 2.4 (t, 2H), 2.75 (t, 2H), 3.18 (t, 2H), 6.42 (d, 1H), 7.65 (d, 1H), 16.5 (s, 1H) |
| 1-20 | Br | H | nBu | 0.92 (t, 3H), 1.39 (m, 2H), 1.6 (m, 2H), 2.05 (m, 2H), 2.41 (m, 2H), 2.78 (m, 2H), 3.12 (t, 2H), 3.2 (t, 2H), 6.42 (d, 1H), 7.65 (d, 1H), 16.5 (s, 1H) |
| 1-21 | Br | H | CH₂iPr | 0.95 (d, 6H), 1.85 (m, 1H), 2.02 (m, 2H), 2.4 (t, 2H), 2.76 (7, 2H), 3.02 (m, 2H), 5.98 (s, 1H), 6.4 (d, 2H), 7.62 (d, 2H), 16.48 (s, 1H) |
| 1-22 | Br | H | CH₂cPr | 0.25 (m, 2H), 0.55 (m, 2H), 1.05 (m, 1H), 2.03 (m, 2H), 2.39 (m, 2H), 2.78 (m, 2H), 3.18 (d, 2H), 6.48 (d, 1H), 7.45 (d, 1H), 16.50 (s, 1H) |
| 1-23 | Br | H | CH₂tBu | 0.98 (s, 9H), 2.03 (m, 2H), 2.39 (m, 2H), 2.78 (m, 2H), 3.12 (d, 2H), 6.42 (d, 1H), 7.45 (d, 1H), 16.50 (s, 1H) |
| 1-24 | Br | H | CH₂CH₂iPr | 0.90 (d, 6H), 1.5 (m, 2H), 1.7 (m, 1H), 2.05 (m, 2H), 2.4 (m, 2H), 2.78 (m, 2H), 3.22 (m, 2H), 5.88 (t, br, 1H), 6.42 (d, 1H), 7.65 (d, 1H), 16.48 (s, 1H) |
| 1-25 | Br | H | CH₂CH₂OMe | |
| 1-26 | Br | H | CH₂CH₂OEt | |
| 1-27 | Br | H | CH₂CH₂CH₂OMe | |
| 1-28 | Br | H | CH₂CH₂CH₂OMe | |
| 1-29 | F | H | H | δ [DMSO] = 1.9 (m, 2H), 2.38 (m, 2H), 2.65 (m, 2H), 6.38 (dd, 1H), 7.4 (dd, 1H), 16.25 (s, br, 1H) |
| 1-30 | F | H | Me | |
| 1-31 | F | Me | Me | δ [MeOD] = 2.05 (m, 2H), 2.62 (m, 4H), 3.12 (s, 6H), 6.92 (d, 2H), 7.88 (dd, 1H) |
| 1-32 | F | H | Et | |
| 1-33 | F | H | nPr | |
| 1-34 | F | H | nBu | |
| 1-35 | F | H | CH₂iPr | |
| 1-36 | F | H | CH₂cPr | |
| 1-37 | F | H | CH₂tBu | |
| 1-38 | F | H | CH₂CH₂iPr | |
| 1-39 | F | H | CH₂CH₂OMe | |
| 1-40 | F | H | CH₂CH₂OEt | |
| 1-41 | F | H | CH₂CH₂CH₂OMe | |
| 1-42 | F | H | CH₂CH₂CH₂OMe | |
| 1-43 | SO₂Me | H | H | 2.05 (m, 2H), 2.4 (t, 2H), 2.8 (t, 2H), 3.15 (s, 3H), 6.52 (d, 1H), 7.62 (s, br, 2H), 8.08 (d, 1H), 16.1 (s, br, 1H) |
| 1-44 | SO₂Me | H | Me | |
| 1-45 | SO₂Me | Me | Me | |
| 1-46 | SO₂Me | H | Et | |
| 1-47 | SO₂Me | H | nPr | |
| 1-48 | SO₂Me | H | nBu | |
| 1-49 | SO₂Me | H | CH₂iPr | |
| 1-50 | SO₂Me | H | CH₂cPr | |
| 1-51 | SO₂Me | H | CH₂tBu | |
| 1-52 | SO₂Me | H | CH₂CH₂iPr | |
| 1-53 | SO₂Me | H | CH₂CH₂OMe | |
| 1-54 | SO₂Me | H | CH₂CH₂OEt | |
| 1-55 | SO₂Me | H | CH₂CH₂CH₂OMe | |
| 1-56 | SO₂Me | H | CH₂CH₂CH₂OMe | |
| 1-57 | SO₂Et | H | H | |
| 1-58 | SO₂Et | H | Me | |
| 1-59 | SO₂Et | Me | Me | |
| 1-60 | SO₂Et | H | Et | |
| 1-61 | SO₂Et | H | nPr | |
| 1-62 | SO₂Et | H | nBu | |
| 1-63 | SO₂Et | H | CH₂iPr | |
| 1-64 | SO₂Et | H | CH₂cPr | |
| 1-65 | SO₂Et | H | CH₂tBu | |
| 1-66 | SO₂Et | H | CH₂CH₂iPr | |
| 1-67 | SO₂Et | H | CH₂CH₂OMe | |
| 1-68 | SO₂Et | H | CH₂CH₂OEt | |
| 1-69 | SO₂Et | H | CH₂CH₂CH₂OMe | |
| 1-70 | SO₂Et | H | CH₂CH₂CH₂OMe | |

**Tabelle 2: Erfindungsgemäße Verbindungen der allgemeinen Formel (I), worin R³, R⁴, R⁶, R⁷ und R⁸ jeweils für Wasserstoff stehen und R⁵ für Methyl steht**

| | | | | |
|---|---|---|---|---|
| 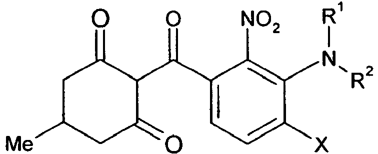 | | | | |

| Nr. | X | R¹ | R² | Physikalische Daten: ¹H-NMR: δ [CDCl₃] |
|---|---|---|---|---|
| 2-1 | Cl | H | H | 1.10 (d, 3H), 2.12 (dd, 1H), 2.28 (m, 1H), 2.4-2.52 (m, 2H), 2.78 (dd, 1H), 6.36 (d, 1H), 6.65 (s, br, 1H), 7.5 (d, 1H), 16.4 (s, 1H) |
| 2-2 | Cl | H | Me | 1.10 (d, 3H), 2.12 (dd, 1H), 2.28 (m, 1H), 2.4-2.52 (m, 2H), 2.78 (dd, 1H), 3.05 (d, 3H), 6.45 (d, 1H), 6.52 (s, br, 1H), 7.45 (d, 1H), 16.42 (s, br, 1H) |
| 2-3 | Cl | Me | Me | 1.10 (d, 3H), 2.12 (dd, 1H), 2.28 (m, 1H), 2.4-2.52 (m, 2H), 2.78 (dd, 1H), 2.85 (s, 6H), 6.85 (d, 1H), 7.5 (d. 1H), 16.4 (s, br, 1H) |
| 2-4 | Cl | H | Et | 1.10 (d, 3H), 1.25 (t, 3H), 2.12 (dd, 1H), 2.28 (m, 1H), 2.4-2.52 (m, 2H), 2.78 (dd, 1H), 3.4 (q, 2H), 6.38 (s, br, 1H), 6.48 (d, 1H), 7.46 (d, 1H), 16.45 (s, br, 1H) |
| 2-5 | Cl | H | nPr | 0.9 (t, 3H), 1.1 (d, 3H), 1.65 (m, 2H), 2.12 (dd, 1H), 2.3 (m, 1H), 2.48 (m, 2H), 2.78 (dd, 1H), 3.32 (t, 2H), 6.45 (d, 1H), 6.5 (s, br, 1H), 7.45 (d, 1H), 16.45 (s, 1H) |
| 2-6 | Cl | H | nBu | 0.92 (t, 3H), 1.10 (d, 3H), 1.39 (m, 2H), 1.6 (m, 2H), 2.12 (dd, 1H), 2.28 (m, 1H), 2.48 (m, 2H), 2.78 (dd, 1H), 3.35 (t, 2H), 6.45 (d, 1H), 7.45 (d, 1H), 16.42 (s, 1 H) |
| 2-7 | Cl | H | CH₂iPr | 0.95 (d, 6H), 1,1 (d, 3H), 1.85 (m, 1H), 2.22 (dd, 1H), 2.43 (dd, 1H), 2.49 (dd, 1H), 2.79 (dd, 1H), 3.02 (d, 2H), 5.95 (s, 1H), 6.4 (d, 1H), 7.62 (d, 1H), 16.42 (s, 1H) |
| 2-8 | Cl | H | CH₂cPr | 0.25 (m, 2H), 0.55 (m, 2H), 1.05 (m, 1H), 1.1 (d, 3H), 2.22 (dd, 2H), 2.28 (m, 1H), 2.48 (m, 2H), 2.78 (dd, 1H), 3.18 (d, 2H), 6.48 (d, 1H), 6.55 (s, br, 1H), 7.45 (d, 1H), 16.45 (s, 1H) |
| 2-9 | Cl | H | CH₂tBu | 0.98 (s, 9H), 1.10 (d, 3H), 2.12 (dd, 1H), 2.28 (m, 1H), 2.48 (m, 2H), 2.78 (dd, 1H), 3.12 (s, 2H), 6.42 (d, 1H), 7.45 (d, 1H), 16.45 (s, 1H) |
| 2-10 | Cl | H | CH₂CH₂iPr | 0.9 (d, 6H), 1.1 (d, 3H), 1.51 (q, 2H), 1.68 (m, 1H), 2.12 (dd, 1H), 2.28 (m, 1H), 2.48 (m, 2H), 2.78 (dd, 1H), 3.35 (t, 2H), 6.45 (s, br, 1H), 6.45 (d, 1H), 7.45 (d, 1H), 16.42 (s, 1H) |
| 2-11 | Cl | H | CH₂CH₂OMe | |
| 2-12 | Cl | H | CH₂CH₂OEt | |
| 2-13 | Cl | H | CH₂CH₂CH₂OMe | 1.1 (d, 3H), 1.85 (m, 2H), 2.12 (dd, 1H), 2.28 (m, 1H), 2.42 (dd, 1H), 2.45 (dd, 1H), 2.78 (dd, 1H), 3.35 (s. 3H), 3.4 (m, 2H), 3.5 (t, 2H), 6.42 (d, 1H), 6.72 (t, br, 1H), 7.42 (d, 1H), 16.45 (s, 1H) |
| 2-14 | Cl | H | CH₂CH₂CH₂OMe | |
| 2-15 | Br | H | H | 1.10 (d, 3H), 2.1 (dd, 1H), 2.28 (m, 1H), 2.4 (dd, 1H), 2.5 (dd, 1H), 2.78 (dd, 1H), 6.3 (d, 1H), 6.3 (s, br, 1H), 7.68 (d, 1H), 16.4 (s, 1H) |
| 2-16 | Br | H | Me | 1.10 (d, 3H), 2.12 (dd, 1H), 2.28 (m, 1H), 2.4-2.52 (m, 2H), 2.78 (dd, 1H), 2.98 (s, 3H), 5.95 (s, br, 1H), 6.4 (d, 1H), 7.65 (d, 1H), 16.42 (s, 1H) |
| 2-17 | Br | Me | Me | |
| 2-18 | Br | H | Et | 1.10 (d, 3H), 1.25 (t, 3H), 2.12 (dd, 1H), 2.28 (m, 1H), 2.4-2.52 (m, 2H), 2.78 (dd, 1H), 3.5 (m, br, 2H), 5.79 (s, br, 1H), 6.42 (d, 1H), 7.65 (d. 1H), 16.42 (s, 1H) |
| 2-19 | Br | H | nPr | 0.9 (t, 3H), 1.1 (d, 3H), 1.65 (m, 2H), 2.12 (dd, 1H), 2.3 (m, 1H), 2.48 (m, 2H), 2.78 (dd, 1H), 3.18 (t, br, 2H), 5.88 (s, br, 1H), 6.42 (d, 1H), 7.65 (d, 1H), 16.42 (s, 1H) |
| 2-20 | Br | H | nBu | 0.92 (t, 3H), 1.10 (d, 3H), 1.39 (m, 2H), 1.6 (m, 2H), 2.12 (dd, 1H), 2.3 (m, 1H), 2.48 (m, 2H), 2.78 (dd, 1H), 3.2 (t, 2H), 6.42 (d, 1H), 7.65 (d, 1H), 16.42 (s, 1H) |
| 2-21 | Br | H | CH₂iPr | 0.95 (d, 6H), 1,1 (d, 3H), 1.85 (m, 1H), 2.22 (dd, 1H), 2.43 (dd, 1H), 2.49 (dd, 1H), 2.79 (dd, 1H), 3.02 (d, 2H), 5.95 (s, 1H), 6.4 (d, 1H), 7.62 (d, 1H), 16.42 (s, 1H) |
| 2-22 | Br | H | CH₂cPr | 0.25 (m, 2H), 0.55 (m, 2H), 1.05 (m, 1H), 1.1 (d, 3H), 2.22 (dd, 2H), 2.28 (m, 1H), 2.48 (m, 2H), 2.78 (dd, 1 H), 3.18 (d, 2H), 6.48 (d, 1H), 7.45 (d, 1H), 16.45 (s, 1H) |
| 2-23 | Br | H | CH₂tBu | 0.98 (s, 9H), 1.10 (d, 3H), 2.12 (dd, 1H), 2.28 (m, 1H), 2.48 (m, 2H), 2.78 (dd, 1H), 3.12 (s, 2H), 6.42 (d, 1H), 7.45 (d, 1H), 16.45 (s, 1H) |
| 2-24 | Br | H | CH₂CH₂iPr | 0.9 (d, 6H), 1.1 (d, 3H), 1.51 (q, 2H), 1.68 (m, 1H), 2.12 (dd, 1H), 2.28 (m, 1H), 2.48 (m, 2H), 2.78 (dd, 1H), 3.22 (t, 2H), 5.85 (s, br, 1H), 6.42 (d, 1H), 7.65 (d, 1H), 16.42 (s, 1H) |
| 2-25 | Br | H | CH₂CH₂OMe | |
| 2-26 | Br | H | CH₂CH₂OEt | |
| 2-27 | Br | H | CH₂CH₂CH₂OMe | |
| 2-28 | Br | H | CH₂CH₂CH₂OMe | |
| 2-29 | F | H | H | |
| 2-30 | F | H | Me | |
| 2-31 | F | Me | Me | |
| 2-32 | F | H | Et | |
| 2-33 | F | H | nPr | |
| 2-34 | F | H | nBu | |
| 2-35 | F | H | CH₂iPr | |
| 2-36 | F | H | CH₂cPr | |
| 2-37 | F | H | CH₂tBu | |
| 2-38 | F | H | CH₂CH₂iPr | |
| 2-39 | F | H | CH₂CH₂OMe | |
| 2-40 | F | H | CH₂CH₂OEt | |
| 2-41 | F | H | CH₂CH₂CH₂OMe | |
| 2-42 | F | H | CH₂CH₂CH₂OMe | |
| 2-43 | SO₂Me | H | H | |
| 2-44 | SO₂Me | H | Me | |
| 2-45 | SO₂Me | Me | Me | |
| 2-46 | SO₂Me | H | Et | |
| 2-47 | SO₂Me | H | nPr | |
| 2-48 | SO₂Me | H | nBu | |
| 2-49 | SO₂Me | H | CH₂iPr | |
| 2-50 | SO₂Me | H | CH₂cPr | |
| 2-51 | SO₂Me | H | CH₂tBu | |
| 2-52 | SO₂Me | H | CH₂CH₂iPr | |
| 2-53 | SO₂Me | H | CH₂CH₂OMe | |
| 2-54 | SO₂Me | H | CH₂CH₂OEt | |
| 2-55 | SO₂Me | H | CH₂CH₂CH₂OMe | |
| 2-56 | SO₂Me | H | CH₂CH₂CH₂OMe | |
| 2-57 | SO₂Et | H | H | |
| 2-58 | SO₂Et | H | Me | |
| 2-59 | SO₂Et | Me | Me | |
| 2-60 | SO₂Et | H | Et | |
| 2-61 | SO₂Et | H | nPr | |
| 2-62 | SO₂Et | H | nBu | |
| 2-63 | SO₂Et | H | CH₂iPr | |
| 2-64 | SO₂Et | H | CH₂cPr | |
| 2-65 | SO₂Et | H | CH₂tBu | |
| 2-66 | SO₂Et | H | CH₂CH₂iPr | |
| 2-67 | SO₂Et | H | CH₂CH₂OMe | |
| 2-68 | SO₂Et | H | CH₂CH₂OEt | |
| 2-69 | SO₂Et | H | CH₂CH₂CH₂OMe | |
| 2-70 | SO₂Et | H | CH₂CH₂CH₂OMe | |

**Tabelle 3: Erfindungsgemäße Verbindungen der allgemeinen Formel (I), worin R⁵, R⁶, R⁷und R⁸ jeweils für Wasserstoff und R³ und R⁴ für jeweils Methyl stehen**

| | | | | |
|---|---|---|---|---|
| 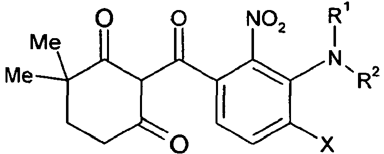 | | | | |

| Nr. | X | R¹ | R² | Physikalische Daten: ¹H-NMR: δ [CDCl₃] |
|---|---|---|---|---|
| 3-1 | Cl | H | H | 1.10 und 1.38 (s, 6H), 1.85 (m, 2H), 2.4 und 2.78 (m, 2H), 6.45 und 6.46 (d, 1H), 7.51 (d,d 1H), 16.32 und 17 (s, 1H) |
| 3-2 | Cl | H | Me | 1.10 und 1.38 (s, 6H), 1.85 (m, 2H), 2.43 und 2.78 (m, 2H), 3.04 und 3.05 (s, 3H), 6.42 und 6.44 (d, 1H), 7.45 (d,d 1H), 16.32 und 17 (s, 1H) |
| 3-3 | Cl | Me | Me | 1.10 und 1.38 (s, 6H), 1.85 (m, 2H), 2.43 und 2.78 (m, 2H), 2.85 (s, 6H), 6.85 und 6.88 (d, 1H), 7.5 (d, 1H), 16.3 und 16.9 (s, 1H) |
| 3-4 | Cl | H | Et | 1.10 und 1.38 (s, 6H), 1.25 (m, 3H), 1.85 (m, 2H), 2.43 und 2.78 (m, 2H), 3.38 (m, 2H), 6.25 und 6.32 (s, br, 1H), 6.46 (d,d 1H), 7.45 (d,d 1H), 16.32 und 16.98 (s, br, 1H) |
| 3-5 | Cl | H | nPr | 0.96 (t, 3H), 1.08 und 1.38 (s, 6H), 1.62 (m, 2H), 1.85 (m, 2H), 2.45 und 2.78 (m, 2H), 3.30 (m, 2H), 6.45 (d,d 1H), 7.45 (d,d, 1H), 16.35 und 17.0 (s, br, 1H) |
| 3-6 | Cl | H | nBu | 0.92 (t, 3H), 1.10 und 1.38 (s, 6H), 1.39 (m, 2H), 1.6 (m, 2H), 1.85 (m, 2H), 2.43 und 2.78 (m, 2H), 3.32 (m, 2H), 6.45 (d,d 1H), 7.45 (d,d 1H), 16.35 und 17.0 (s, 1H) |
| 3-7 | Cl | H | CH₂iPr | 0.95 (d, 6H), 1.10 und 1.38 (s, 6H), 1.85 (m, 1H), 1.85 und 2.43 (m, 2H), 2.78 (m, 2H), 3.15 und 3.18 (d, 2H), 6.45 (d,d, 1H), 7.45 (d,d, 1H), 16.35 und 17.0 (s, 1H) |
| 3-8 | Cl | H | CH₂cPr | 0.25 (m, 2H), 0.55 (m, 2H), 1.05 (m, 1H), 1.10 und 1.38 (s, 6H), 1.85 (m, 2H), 2.43 und 2.78 (t, 2H), 3.15 (m, 2H), 6.4 und 6.48 (s, br, 1H), 6.48 (d,d 1H), 7.45 (d,d 1H), 16.35 und 16.98 (s, 1H) |
| 3-9 | Cl | H | CH₂tBu | 0.98 (s, 9H), 1.10 und 1.38 (s, 6H), 1.85 (m, 2H), 2.43 und 2.78 (m, 2H), 3.1 (m, 2H), 6.4 und 6.48 (s, br, 1H), 6.43 (d,d 1H), 7.45 (d,d 1H), 16.35 und 17.0 (s, 1H) |
| 3-10 | Cl | H | CH₂CH₂iPr | 0.92 (d, 6H), 1.10 und 1.38 (s, 6H), 1.52 (m, 2H), 1.65 (m, 1H), 1.85 (m, 2H), 2.43 und 2.78 (m, 2H), 3.35 (m, 2H), 6.3 und 6.4 (t, br, 1H), 6.45 (d,d 1H), 7.45 (d,d 1H), 16.35 und 16.98 (s, 1H) |
| 3-11 | Cl | H | CH₂CH₂OMe | |
| 3-12 | Cl | H | CH₂CH₂OEt | |
| 3-13 | Cl | H | CH₂CH₂CH₂OMe | |
| 3-14 | Cl | H | CH₂CH₂CH₂OMe | |
| 3-15 | Br | H | H | 1.09 und 1.38 (s, 6H), 1.85 (m, 2H), 2.4 und 2.78 (m, 2H), 6.3 (d,d 1H), 6.7 (s, br, 2H), 7.68 (d,d 1H), 16.3 und 16.98 (s, br, 1H) |
| 3-16 | Br | H | Me | 1.10 und 1.38 (s, 6H), 1.85 (m, 2H), 2.43 und 2.78 (m, 2H), 2.95 (d, 3H), 6.4 (d,d 1H), 7.62 (d,d 1H), 16.32 und 16.98 (s, br, 1H) |
| 3-17 | Br | Me | Me | δ [MeOD] = 1.2 (s, 6H), 1.9 (t, 2H), 2.75 (m, 2H), 2.85 (s, 6H), 6.95 (d, 1H), 7.8 (d, 1H) |
| 3-18 | Br | H | Et | 1.10 und 1.38 (s, 6H), 1.25 (m, 3H), 1.85 (m, 2H), 2.43 und 2.78 (m, 2H), 3.25 (m, 2H), 5.65 und 5.75 (s, br, 1H), 6.42 (d,d 1H), 7.65 (d,d 1H), 16.32 und 16.98 (s, br, 1H) |
| 3-19 | Br | H | nPr | 0.96 (t, 3H), 1.1 und 1.38 (s, 6H), 1.62 (m, 2H), 1.85 (m, 2H), 2.43 und 2.78 (m, 2H), 3.15 (m, 2H), 5.75 und 5.85 (s, br, 1H) 6.42 (d,d 1H), 7.62 (d,d, 1H), 16.35 und 16.98 (s, br, 1H) |
| 3-20 | Br | H | nBu | 0.92 (t, 3H), 1.10 und 1.38 (s, 6H), 1.39 (m, 2H), 1.6 (m, 2H), 1.85 (m, 2H), 2.43 und 2.78 (m, 2H), 3.18 (t, 2H), 6.4 (d,d 1H), 7.65 (d,d 1H), 16.35 und 17.0 (s, 1H) |
| 3-21 | Br | H | CH₂iPr | 0.95 (d, 6H), 1.10 und 1.38 (s, 6H), 1.85 (m, 1H), 1.85 und 2.43 (m, 2H), 2.78 - 3.0 (m, 2H), 2.99 (m, 2H), 5.82 und 5.9 (t, br, 1H), 6.4 (d,d, 1H), 7.65 (d,d, 1H), 16.45 und 16.98 (s, 1H) |
| 3-22 | Br | H | CH₂cPr | 0.25 (m, 2H), 0.55 (m, 2H), 1.05 (m, 1H), 1.10 und 1.38 (s, 6H), 1.85 und 2.43 (m, 2H), 2.78 - 2.95 (m, 2H), 3.02 (m, 2H), 6.48 (d,d 1H), 7.65 (d,d 1H), 16.35 und 16.98 (s, 1H) |
| 3-23 | Br | H | CH₂tBu | 0.98 (s, 9H), 1.10 und 1.38 (s, 6H), 1.85 (m, 2H), 2.43 und 2.78 (m, 2H), 2.85 - 2.95 (m, 2H), 5.78 und 5.88 (t, br, 1H), 6.4 (d,d 1H), 7.65 (d,d 1H), 16.35 und 17.0 (s, 1H) |
| 3-24 | Br | H | CH₂CH₂iPr | 0.92 (d, 6H), 1.10 und 1.38 (s, 6H), 1.5 (m, 2H), 1.65 (m, 1H), 1.85 (m, 2H), 2.43 und 2.78 (m, 2H), 3.18 (m, 2H), 6.41 (d,d 1H), 7.63 (d,d 1H), 16.35 und 16.98 (s, 1H) |
| 3-25 | Br | H | CH₂CH₂OMe | |
| 3-26 | Br | H | CH₂CH₂OEt | |
| 3-27 | Br | H | CH₂CH₂CH₂OMe | |
| 3-28 | Br | H | CH₂CH₂CH₂OMe | |
| 3-29 | F | H | H | 1.08 und 1.38 (s, 3H), 1.85 (m, 2H), 2.4 und 2.78 (t, 2H), 6.2 (s, br, 2H), 6.32 (m, 1H), 7.2 (m, 1H), 16.42 und 17.1 (s, 1H) |
| 3-30 | F | H | Me | |
| 3-31 | F | Me | Me | |
| 3-32 | F | H | Et | |
| 3-33 | F | H | nPr | |
| 3-34 | F | H | nBu | |
| 3-35 | F | H | CH₂iPr | |
| 3-36 | F | H | CH₂cPr | |
| 3-37 | F | H | CH₂tBu | |
| 3-38 | F | H | CH₂CH₂iPr | |
| 3-39 | F | H | CH₂CH₂OMe | |
| 3-40 | F | H | CH₂CH₂OEt | |
| 3-41 | F | H | CH₂CH₂CH₂OMe | |
| 3-42 | F | H | CH₂CH₂CH₂OMe | |
| 3-43 | SO₂Me | H | H | 1.02 und 1.32 (s, 3H), 1.8 (m, 2H), 2.38 und 2.75 (t, 2H), 3.07 und 3.08 (s, 3H), 6.42 und 6.46 (d, 2H), 7.52 (s, 2H), 8.01 (d, 2H), 15.89 und 16.58 (s, 1H) |
| 3-44 | SO₂Me | H | Me | δ [MeOD] = 1.2 (s, 6H), 1.8 (t, 2H), 2.32 (t, 2H), 2.68 (s, 3H), 3.1 (s, 3H), 6.51 (d, 1H), 7.85 (d, 1H) |
| 3-45 | SO₂Me | Me | Me | |
| 3-46 | SO₂Me | H | Et | |
| 3-47 | SO₂Me | H | nPr | |
| 3-48 | SO₂Me | H | nBu | |
| 3-49 | SO₂Me | H | CH₂iPr | |
| 3-50 | SO₂Me | H | CH₂cPr | |
| 3-51 | SO₂Me | H | CH₂tBu | |
| 3-52 | SO₂Me | H | CH₂CH₂iPr | |
| 3-53 | SO₂Me | H | CH₂CH₂OMe | |
| 3-54 | SO₂Me | H | CH₂CH₂OEt | |
| 3-55 | SO₂Me | H | CH₂CH₂CH₂OMe | |
| 3-56 | SO₂Me | H | CH₂CH₂CH₂OMe | |
| 3-57 | SO₂Et | H | H | |
| 3-58 | SO₂Et | H | Me | |
| 3-59 | SO₂Et | Me | Me | |
| 3-60 | SO₂Et | H | Et | |
| 3-61 | SO₂Et | H | nPr | |
| 3-62 | SO₂Et | H | nBu | |
| 3-63 | SO₂Et | H | CH₂iPr | |
| 3-64 | SO₂Et | H | CH₂cPr | |
| 3-65 | SO₂Et | H | CH₂tBu | |
| 3-66 | SO₂Et | H | CH₂CH₂iPr | |
| 3-67 | SO₂Et | H | CH₂CH₂OMe | |
| 3-68 | SO₂Et | H | CH₂CH₂OEt | |
| 3-69 | SO₂Et | H | CH₂CH₂CH₂OMe | |
| 3-70 | SO₂Et | H | CH₂CH₂CH₂OMe | |

**Tabelle 4: Erfindungsgemäße Verbindungen der allgemeinen Formel (I), worin R⁴, R⁵, R⁶ und R⁷ jeweils für Wasserstoff stehen und R³ und R⁸ für CH₂CH₂ stehen**

| | | | | |
|---|---|---|---|---|
| 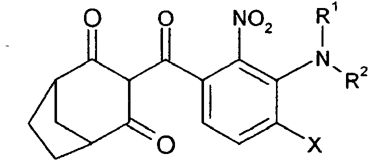 | | | | |

| Nr. | X | R¹ | R² | Physikalische Daten: ¹H-NMR: δ [CDCl₃] |
|---|---|---|---|---|
| 4-1 | Cl | H | H | 1.68 (m, 2H), 1.98 - 2.25 (m, 4H), 2.82 (m, 1H), 3.1 (m, 1H), 6.39 (s, 1H), 6.62 (s, br, 2H), 7.51 (d, 1H), 16.3 (s, 1H) |
| 4-2 | Cl | H | Me | 1.7 (m, 2H), 1.98 - 2.25 (m, 4H), 2.85 (m, 1H), 3.05 (s, 3H), 3.12 (m, 1H), 6.49 (d, 1H), 7.46 (d, 1H), 16.38 (s, 1H) |
| 4-3 | Cl | Me | Me | 1.68 (m, 2H), 1.98 - 2.25 (m, 4H), 2.82 (s, 6H), 2.89 (m, 1H), 3.12 (m, 1H), 6.9 (d, 1H), 7.5 (d, 1H), 16.4 (s, 1H) |
| 4-4 | Cl | H | Et | 1.25 (t, 3H), 1.68 (m, 2H), 1.98 - 2.25 (m, 4H), 2.89 (m, 1H), 3.12 (m, 1H), 3.48 (q, 3H), 6.5 (d, 1H), 7.485 (d, 1H), 16.4 (s, 1H) |
| 4-5 | Cl | H | nPr | 0.95 (t, 3H), 1.65 (q, 2H), 1.7 (m, 2H), 1.98-2.25 (m, 4H), 2.85 (m, 1H), 3.1 (m, 1H), 3.3 (t, 2H), 6.38 (s, 1H), 6.5 (d, 1H), 7.46 (d, 1H), 16.4 (s, br, 1H) |
| 4-6 | Cl | H | nBu | 0.92 (t, 3H), 1.38 (m, 2H), 1.6 (m, 2H), 1.7 (m, 2H), 1.98 - 2.25 (m, 4H), 2.85 (m, 1H), 3.1 (m, 1H), 3.33 (q, 2H), 6.48 (d, 1H), 7.45 (d, 1H), 16.38 (s, br, 1H) |
| 4-7 | Cl | H | CH₂iPr | 0.98 (d, 6H), 1.7 (m, 2H), 1.85 (m, 1H), 1.98 - 2.25 (m, 4H), 2.85 (m, 1H), 3.1 (m, 1H), 3.15 (d, 2H), 6.48 (d, 1H), 7.45 (d, 1H), 16.4 (s, 1H) |
| 4-8 | Cl | H | CH₂cPr | 0.25 (m, 2H), 0.55 (m, 2H), 1.05 (m, 1H), 1.7 (m, 2H), 1.98 - 2.25 (m, 4H), 2.85 (m, 1H), 3.1 (m, 1H), 3.15 (d, 2H), 6.4 (s, br, 1H), 6.5 (d, 1H), 7.45 (d, 1H), 16.38 (s, 1H) |
| 4-9 | Cl | H | CH₂tBu | 0.98 (s, 9H), 1.7 (m, 2H), 1.98 - 2.25 (m, 4H), 2.85 (m, 1H), 3.1 (m, 1H), 6.48 (d, 1H), 7.45 (d, 1H), 16.38 (s, 1H) |
| 4-10 | Cl | H | CH₂CH₂iPr | 0.90 (d, 6H), 1.5 (m, 2H), 1.7 (m, 1H), 1.7 (m, 2H), 1.98 - 2.25 (m, 4H), 2.85 (m, 1H), 3.1 (m, 1H), 3.35 (m, 2H), 6.35 (s, br, 1H), 6.5 (d, 1H), 7.45 (d, 1H), 16.38 (s, 1H) |
| 4-11 | Cl | H | CH₂CH₂OMe | |
| 4-12 | Cl | H | CH₂CH₂OEt | |
| 4-13 | Cl | H | CH₂CH₂CH₂OMe | 1.7 (m, 2H), 1.85 (m, 2H), 1.98 - 2.25 (m, 4H), 2.85 (m, 1H), 3.1 (m, 1H), 3.5 (s, 3H), 3.8 (t, 2H), 3.48 (m, 2H), 6.48 (d, 1H), 7.45 (d, 1H), 16.4 (s, br, 1H) |
| 4-14 | Cl | H | CH₂CH₂CH₂OMe | |
| 4-15 | Br | H | H | 1.7 (m, 2H), 1.98 - 2.25 (m, 4H), 2.81 (m, 1H), 3.1 (m, 1H), 6.35 (d, 1H), 6.68 (s, br, 2H), 7.68 (d, 1H), 16.28 (s, 1H) |
| 4-16 | Br | H | Me | 1.72 (m, 2H), 1.98 - 2.25 (m, 4H), 2.88 (m, 1H), 2.95 (s, 3H), 3.12 (m, 1H), 6.42 (d, 1H), 7.62 (d, 1H), 16.38 (s, br, 1H) |
| 4-17 | Br | Me | Me | 1.72 (m, 2H), 1.98 - 2.25 (m, 4H), 2.82 (s, 6H), 2.9 (m, 1H), 3.12 (m, 1H), 6.82 (d, 1H), 7.72 (d, 1H), 16.4 (s, br, 1H) |
| 4-18 | Br | H | Et | 1.25 (t, 3H), 1.7 (m, 2H), 1.98 - 2.25 (m, 4H), 2.85 (m, 1H), 3.1 (m, 1H), 3.25 (q, 2H), 5.7 (s, br, 1H), 6.48 (d, 1H), 7.65 (d, 1H), 16.38 (s, br, 1H) |
| 4-19 | Br | H | nPr | 0.98 (t, 3H), 1.62 (m, 2H), 1.7 (m, 2H), 1.98 - 2.25 (m, 4H), 2.85 (m, 1H), 3.1 (m, 1H), 3.15 (t, 2H), 5.8 (s, br, 1H), 6.48 (d, 1H), 7.65 (d, 1H), 16.38 (s, br, 1H) |
| 4-20 | Br | H | nBu | 0.92 (t, 3H), 1.38 (m, 2H), 1.6 (m, 2H), 1.7 (m, 2H), 1.98 - 2.25 (m, 4H), 2.85 (m, 1H), 3.1 (m, 1H), 3.28 (q, 2H), 5.78 (s, br, 1H), 6.45 (d, 1H), 7.65 (d, 1H), 16.38 (s, br, 1H) |
| 4-21 | Br | H | CH₂iPr | 0.98 (d, 6H), 1.7 (m, 2H), 1.85 (m, 1H), 1.98 - 2.25 (m, 4H), 2.85 (m, 1H), 3.0 (d, 2H), 3.1 (m, 1H), 5.7 (s, br, 1H), 6.45 (d, 1H), 7.65 (d, 1H), 16.38 (s, 1H) |
| 4-22 | Br | H | CH₂cPr | 0.25 (m, 2H), 0.55 (m, 2H), 1.05 (m, 1H), 1.7 (m, 2H), 1.98 - 2.25 (m, 4H), 2.85 (m, 1H), 3.1 (m, 1H), 3.15 (d, 2H), 6.4 (s, br, 1H), 6.5 (d, 1H), 7.45 (d, 1H), 16.38 (s, 1H) |
| 4-23 | Br | H | CH₂tBu | 0.98 (s, 9H), 1.7 (m, 2H), 1.98 - 2.25 (m, 4H), 2.85 (m, 1H), 3.1 (m, 1H), 6.48 (d, 1H), 7.45 (d, 1H), 16.4 (s, 1H) |
| 4-24 | Br | H | CH₂CH₂iPr | 0.90 (d, 6H), 1.5 (m, 2H), 1.7 (m, 1H), 1.7 (m, 2H), 1.98 - 2.25 (m, 4H), 2.85 (m, 1H), 3.1 (m, 1H), 3.22 (m, 2H), 5.88 (t, br, 1H), 6.42 (d, 1H), 7.65 (d, 1H), 16.48 (s, 1H) |
| 4-25 | Br | H | CH₂CH₂OMe | |
| 4-26 | Br | H | CH₂CH₂OEt | |
| 4-27 | Br | H | CH₂CH₂CH₂OMe | |
| 4-28 | Br | H | CH₂CH₂CH₂OMe | |
| 4-29 | F | H | H | 1.7 (m, 2H), 1.98 - 2.25 (m, 4H), 2.82 (m, 1H), 3.1 (m, 1H), 6.2 (s, br, 2H), 6.38 (dd, 1H), 7.2 (dd, 1H), 16.42 (s, 1H) |
| 4-30 | F | H | Me | δ [MeOD] = 1.65 (m, 6H), 2.82 (m, 2H), 2.95 (s, 3H), 6.78 (d, 1H), 7.88 (dd, 1H) |
| 4-31 | F | Me | Me | δ [MeOD] = 1.4 (m, 2H), 1.65 (m, 4H), 2.85 (m, 2H), 3.12 (s, 6H), 6.92 (dd, 1H), 7.88 (dd, 1H) |
| 4-32 | F | H | Et | |
| 4-33 | F | H | nPr | |
| 4-34 | F | H | nBu | |
| 4-35 | F | H | CH₂iPr | |
| 4-36 | F | H | CH₂cPr | |
| 4-37 | F | H | CH₂tBu | |
| 4-38 | F | H | CH₂CH₂iPr | |
| 4-39 | F | H | CH₂CH₂OMe | |
| 4-40 | F | H | CH₂CH₂OEt | |
| 4-41 | F | H | CH₂CH₂CH₂OMe | |
| 4-42 | F | H | CH₂CH₂CH₂OMe | |
| 4-43 | SO₂Me | H | H | 1.7 (m, 2H), 1.98 - 2.25 (m, 4H), 2.85 (m, 1H), 3.1 (m, 1H), 3.15 (s, 3H), 6.55 (d, 1H), 8.09 (d, 1H), 15.92 (s, br, 1H) |
| 4-44 | SO₂Me | H | Me | δ [MeOD] = 1.4 (m, 2H), 1.65 (m, 4H), 2.85 (m, 2H), 2.88 (s, 3H), 3.18 (s, 3H), 7.25 (d, 1H), 7.98 (d, 1H) |
| 4-45 | SO₂Me | Me | Me | 1.7 (m, 2H), 1.98 - 2.25 (m, 4H), 2.85 (m, 1H), 2.86 (s, 6H), 3.1 (m, 1H), 3.15 (s, 3H), 6.55 (d, 1H), 8.09 (d, 1H), 15.92 (s, br, 1H) |
| 4-46 | SO₂Me | H | Et | |
| 4-47 | SO₂Me | H | nPr | |
| 4-48 | SO₂Me | H | nBu | |
| 4-49 | SO₂Me | H | CH₂iPr | |
| 4-50 | SO₂Me | H | CH₂cPr | |
| 4-51 | SO₂Me | H | CH₂tBu | |
| 4-52 | SO₂Me | H | CH₂CH₂iPr | |
| 4-53 | SO₂Me | H | CH₂CH₂OMe | |
| 4-54 | SO₂Me | H | CH₂CH₂OEt | |
| 4-55 | SO₂Me | H | CH₂CH₂CH₂OMe | |
| 4-56 | SO₂Me | H | CH₂CH₂CH₂OMe | |
| 4-57 | SO₂Et | H | H | |
| 4-58 | SO₂Et | H | Me | |
| 4-59 | SO₂Et | Me | Me | |
| 4-60 | SO₂Et | H | Et | |
| 4-61 | SO₂Et | H | nPr | |
| 4-62 | SO₂Et | H | nBu | |
| 4-63 | SO₂Et | H | CH₂iPr | |
| 4-64 | SO₂Et | H | CH₂cPr | |
| 4-65 | SO₂Et | H | CH₂tBu | |
| 4-66 | SO₂Et | H | CH₂CH₂iPr | |
| 4-67 | SO₂Et | H | CH₂CH₂OMe | |
| 4-68 | SO₂Et | H | CH₂CH₂OEt | |
| 4-69 | SO₂Et | H | CH₂CH₂CH₂OMe | |
| 4-70 | SO₂Et | H | CH₂CH₂CH₂OMe | |

**Tabelle 5: Erfindungsgemäße Verbindungen der allgemeinen Formel (I), worin R³, R⁴, R⁷ und R⁸ jeweils für Methyl und R⁵ und R⁶ für =O stehen**

| | | | | |
|---|---|---|---|---|
| 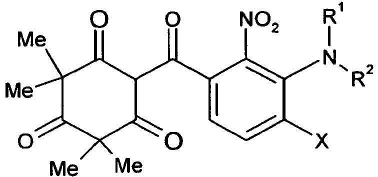 | | | | |

| Nr. | X | R¹ | R² | Physikalische Daten: ¹H-NMR: δ [CDCl₃] |
|---|---|---|---|---|
| 5-1 | Cl | H | H | 1.28 (s, 6H), 1.55 (s, 6H), 6.42 (d, 1H), 6.68 (s, br, 1H), 7.55 (d, 1H), 16.9 (s, 1H) |
| 5-2 | Cl | H | Me | 1.3 (s, 6H), 1.55 (s, 6H), 3.08 (s, 3H), 6.48 (d, 1H), 7.48 (d, 1H), 16.9 (s, 1H) |
| 5-3 | Cl | Me | Me | 1.34 (s, 6H), 1.55 (s, 6H), 2.85 (s, 6H), 6.88 (d, 1H), 7.52 (d, 1H), 16.9 (s, 1H) |
| 5-4 | Cl | H | Et | |
| 5-5 | Cl | H | nPr | |
| 5-6 | Cl | H | nBu | |
| 5-7 | Cl | H | CH₂iPr | |
| 5-8 | Cl | H | CH₂cPr | |
| 5-9 | Cl | H | CH₂tBu | |
| 5-10 | Cl | H | CH₂CH₂iPr | |
| 5-11 | Cl | H | CH₂CH₂OMe | |
| 5-12 | Cl | H | CH₂CH₂OEt | |
| 5-13 | Cl | H | CH₂CH₂CH₂OMe | |
| 5-14 | Cl | H | CH₂CH₂CH₂OMe | |
| 5-15 | Br | H | H | |
| 5-16 | Br | H | Me | |
| 5-17 | Br | Me | Me | |
| 5-18 | Br | H | Et | |
| 5-19 | Br | H | nPr | |
| 5-20 | Br | H | nBu | |
| 5-21 | Br | H | CH₂iPr | |
| 5-22 | Br | H | CH₂cPr | |
| 5-23 | Br | H | CH₂tBu | |
| 5-24 | Br | H | CH₂CH₂iPr | |
| 5-25 | Br | H | CH₂CH₂OMe | |
| 5-26 | Br | H | CH₂CH₂OEt | |
| 5-27 | Br | H | CH₂CH₂CH₂OMe | |
| 5-28 | Br | H | CH₂CH₂CH₂OMe | |
| 5-29 | F | H | H | |
| 5-30 | F | H | Me | |
| 5-31 | F | Me | Me | |
| 5-32 | F | H | Et | |
| 5-33 | F | H | nPr | |
| 5-34 | F | H | nBu | |
| 5-35 | F | H | CH₂iPr | |
| 5-36 | F | H | CH₂cPr | |
| 5-37 | F | H | CH₂tBu | |
| 5-38 | F | H | CH₂CH₂iPr | |
| 5-39 | F | H | CH₂CH₂OMe | |
| 5-40 | F | H | CH₂CH₂OEt | |
| 5-41 | F | H | CH₂CH₂CH₂OMe | |
| 5-42 | F | H | CH₂CH₂CH₂OMe | |
| 5-43 | SO₂Me | H | H | |
| 5-44 | SO₂Me | H | Me | |
| 5-45 | SO₂Me | Me | Me | |
| 5-46 | SO₂Me | H | Et | |
| 5-47 | SO₂Me | H | nPr | |
| 5-48 | SO₂Me | H | nBu | |
| 5-49 | SO₂Me | H | CH₂iPr | |
| 5-50 | SO₂Me | H | CH₂cPr | |
| 5-51 | SO₂Me | H | CH₂tBu | |
| 5-52 | SO₂Me | H | CH₂CH₂iPr | |
| 5-53 | SO₂Me | H | CH₂CH₂OMe | |
| 5-54 | SO₂M | H | CH₂CH₂OEt | |
| 5-55 | SO₂Me | H | CH₂CH₂CH₂OMe | |
| 5-56 | SO₂Me | H | CH₂CH₂CH₂OMe | |
| 5-57 | SO₂Et | H | H | |
| 5-58 | SO₂Et | H | Me | |
| 5-59 | SO₂Et | Me | Me | |
| 5-60 | SO₂Et | H | Et | |
| 5-61 | SO₂Et | H | nPr | |
| 5-62 | SO₂Et | H | nBu | |
| 5-63 | SO₂Et | H | CH₂iPr | |
| 5-64 | SO₂Et | H | CH₂cPr | |
| 5-65 | SO₂Et | H | CH₂tBu | |
| 5-66 | SO₂Et | H | CH₂CH₂iPr | |
| 5-67 | SO₂Et | H | CH₂CH₂OMe | |
| 5-68 | SO₂Et | H | CH₂CH₂OEt | |
| 5-69 | SO₂Et | H | CH₂CH₂CH₂OMe | |
| 5-70 | SO₂Et | H | CH₂CH₂CH₂OMe | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

1. Herbizide Wirkung gegen Schadpflanzen im Vorauflauf Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-1, 4-1, 1-2, 4-2, 3-1, 1-3, 3-2, 3-15, 4-15, 1-15, 1-16, 3-16, 4-16, 1-16, 4-17, 4-29, 1-43, 3-43, 4-43, 5-2, 5-3, 4-3, 3-44, 2-1, 1-17, 3-17, 4-45, 1-4, 4-4, 2-4, 1-5, 3-5, 4-5, 1-19, 4-19, 4-7, 1-18, 4-21, 1-9, 1-7, 3-8 und 1-8 bei einer Aufwandmenge von 320 g/ha jeweils eine mindestens 90%-ige Wirkung gegen Abuthilon theophrasti und Echinocloa crus galli.
2. Herbizide Wirkung gegen Schadpflanzen im Nachauflauf Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-1, 4-1, 1-2, 3-1, 1-3, 3-2, 3-15, 4-15, 1-15, 3-16, 4-17, 4-29, 1-43, 3-43, 4-43, 2-1, 5-3, 4-3, 2-1, 1-17, 3-17, 4-45, 1-4, 4-4, 2-4, 1-5, 3-5, 4-5, 1-19, 3-19, 4-7, 1-18, 3-18, 4-18, 4-6, 1-7, 1-5, 2-6, 2-15, 4-20, 3-8, 2-8 und 1-8 bei einer Aufwandmenge von 80 g/ha jeweils eine mindestens 80%-ige Wirkung gegen Abuthilon theophrasti und Echinocloa crus galli.

## Patentansprüche

1. Verbindungen der Formel (I) oder deren Salze 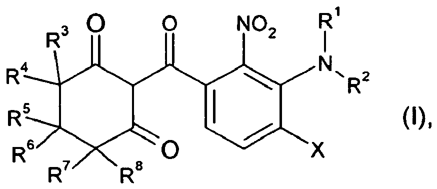 worin
X bedeutet Halogen, Ethylsulfonyl, Methylsulfonyl, 1,2,4-Triazol-1-yl oder 1,2,4-Triazol-4-yl,
R¹ und R² bedeuten unabhängig voneinander jeweils Wasserstoff, (C₁-C₆)-Alkyl, (C₂-C₆)-Alkenyl, (C₂-C₆)-Alkinyl, Halogen-(C₁-C₆)-alkyl, Halogen-(C₁-C₆)-alkenyl, Halogen-(C₁-C₆)-alkinyl, (C₃-C₆)-Cycloalkyl-(C₁-C₆)-alkyl, (C₃-C₆)-Cycloalkenyl-(C₁-C₆)-alkyl, (C₁-C₆)-Alkoxy-(C₁-C₆)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂ oder CH=CH,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

2. Verbindungen nach Anspruch 1, worin
X bedeutet Chlor, Brom, Fluor oder Methylsulfonyl,
R¹ und R² bedeuten unabhängig voneinander jeweils Wasserstoff, (C₁-C₆)-Alkyl, Halogen-(C₁-C₆)-alkyl, Halogen-(C₁-C₆)-Alkenyl, (C₃-C₆)-Cycloalkyl-(C₁-C₆)-alkyl oder (C₁-C₆)-Alkoxy-(C₁-C₆)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

3. Verbindungen nach Anspruch 1 oder 2, worin
X bedeutet Chlor oder Brom,
R¹ bedeutet Wasserstoff oder Methyl,
R² bedeutet Wasserstoff, (C₁-C₄)-Alkyl, Halogen-(C₁-C₄)-alkyl, (C₃-C₆)-Cycloalkyl-(C₁-C₄)-alkyl oder (C₁-C4)-Alkoxy-(C₁-C₄)-alkyl,
R⁴ und R⁷ bedeuten unabhängig voneinander jeweils Wasserstoff oder Methyl,
R³ und R⁸ bedeuten unabhängig voneinander jeweils Wasserstoff oder Methyl, oder R³ und R⁸ bilden gemeinsam die Gruppe CH₂CH₂,
R⁵ und R⁶ bedeuten unabhängig voneinander jeweils Wasserstoff oder Methyl, oder R⁵ und R⁶ bilden gemeinsam mit dem Kohlenstoffatom, an dem sie gebunden sind, die Gruppe C=O.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksame Stoffen aus der Gruppe Insektizide, Akarizide, Herbizide, Fungizide, Safenern und Wachstumsregulatoren.

7. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 6 auf die Pflanzen oder auf den Ort des unerwünschten Pflanzenwachstums appliziert.

8. Verwendung von Verbindungen der Formel (I) gemäß einem der Ansprüche 1 bis 3 oder von herbiziden Mitteln nach einem der Ansprüche 4 bis 6 zur Bekämpfung unerwünschter Pflanzen.

9. Verwendung nach Anspruch 8, **dadurch gekennzeichnet, daß** die Verbindungen der Formel (I) zur Bekämpfung unerwünschter Pflanzen in Kulturen von Nutzpflanzen eingesetzt werden.

10. Verwendung nach Anspruch 9, **dadurch gekennzeichnet, daß** die Nutzpflanzen transgene Nutzpflanzen sind.

## Claims

1. A compound of the formula (I) or salt thereof 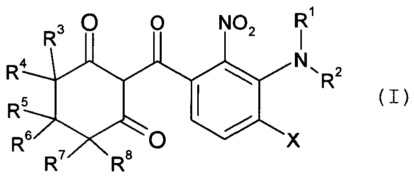 in which
X is halogen, ethylsulfonyl, methylsulfonyl, 1,2,4-triazol-1-yl or 1,2,4-triazol-4-yl,
R¹ and R² are independently of one another each hydrogen, (C₁-C₆) -alkyl, (C₂-C₆)-alkenyl, (C₂-C₆) -alkynyl, halo- (C₁-C₆) -alkyl, halo- (C₁-C₆) -alkenyl, halo- (C₁-C₆) -alkynyl, (C₃-C₆)-cycloalkyl-(C₁-C₆)-alkyl, (C₃-C₆)-cycloalkenyl-(C₁-C₆)-alkyl, (C₁-C₆)-alkoxy-(C₁-C₆)-alkyl,
R⁴ and R⁷ are independently of one another each hydrogen or methyl,
R³ and R⁸ are independently of one another each hydrogen or methyl, or R³ and R⁸ together form the group CH₂CH₂ or CH=CH,
R⁵ and R⁶ are independently of one another each hydrogen or methyl, or R⁵ and R⁶, together with the carbon atom to which they are attached, form the group C=O.

2. The compound as claimed in claim 1, in which
X is chlorine, bromine, fluorine or methylsulfonyl,
R¹ and R² are independently of one another each hydrogen, (C₁-C₆) -alkyl, halo- (C₁-C₆) -alkyl, halo- (C₁-C₆) -alkenyl, (C₃-C₆)-cycloalkyl-(C₁-C₆)-alkyl or (C₁-C₆)-alkoxy-(C₁-C₆)-alkyl,
R⁴ and R⁷ are independently of one another each hydrogen or methyl,
R³ and R⁸ are independently of one another each hydrogen or methyl, or R³ and R⁸ together form the group CH₂CH₂,
R⁵ and R⁶ are independently of one another each hydrogen or methyl, or R⁵ and R⁶, together with the carbon atom to which they are attached, form the group C=O.

3. The compound as claimed in claim 1 or 2, in which
X is chlorine or bromine,
R¹ is hydrogen or methyl,
R² is hydrogen, (C₁-C₄)-alkyl, halo-(C₁-C₄)-alkyl, (C₃-C₆)-cycloalkyl-(C₁-C₄)-alkyl or (C₁-C₄)-alkoxy-(C₁-C₄)-alkyl,
R⁴ and R⁷ are independently of one another each hydrogen or methyl,
R³ and R⁸ are independently of one another each hydrogen or methyl, or R³ and R⁸ together form the group CH₂CH₂,
R⁵ and R⁶ are independently of one another each hydrogen or methyl, or R⁵ and R⁶, together with the carbon atom to which they are attached, form the group C=O.

4. A herbicidal composition which comprises a herbicidally effective amount of at least one compound of the formula (I) as claimed in any of claims 1 to 3.

5. The herbicidal composition as claimed in claim 4 in a mixture with formulation auxiliaries.

6. The herbicidal composition as claimed in claim 4 or 5, comprising at least one further pesticidally active compound from the group of the insecticides, acaricides, herbicides, fungicides, safeners and growth regulators.

7. A method for controlling unwanted plants, which comprises applying an effective amount of at least one compound of the formula (I) as claimed in any of claims 1 to 3 or of a herbicidal composition as claimed in any of claims 4 to 6 to the plants or to the location of the unwanted plant growth.

8. The use of a compound of the formula (I) as claimed in any of claims 1 to 3 or of a herbicidal composition as claimed in any of claims 4 to 6 for controlling unwanted plants.

9. The use as claimed in claim 8, wherein the compounds of the formula (I) are used for controlling unwanted plants in crops of useful plants.

10. The use as claimed in claim 9, wherein the useful plants are transgenic useful plants.

## Revendications

1. Composés de formule (I) ou leurs sels 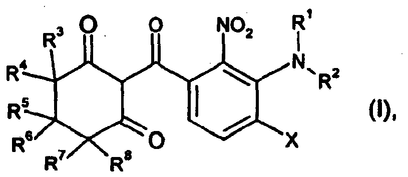 où
X signifie halogène, éthylsulfonyle, méthylsulfonyle, 1,2,4-triazol-1-yle ou 1,2,4-triazol-4-yle,
R¹ et R² signifient, indépendamment l'un de l'autre, à chaque fois hydrogène, (C₁-C₆)-alkyle, (C₂-C₆)-alcényle, (C₂-C₆) -alcynyle, halogéno-(C₁-C₆)-alkyle, halogéno-(C₁-C₆)-alcényle, halogéno-(C₁-C₆)-alcynyle, (C₃-C₆) -cycloalkyl- (C₁-C₆) -alkyle, (C₃-C₆) -cycloalcényl- (C₁-C₆) -alkyle, (C₁-C₆) -alcoxy-(C₁-C₆) -alkyle,
R⁴ et R⁷ signifient, indépendamment l'un de l'autre, à chaque fois hydrogène ou méthyle ;
R³ et R⁸ signifient, indépendamment l'un de l'autre, à chaque fois hydrogène ou méthyle, ou
R³ et R⁸ forment ensemble le groupe CH₂CH₂ ou CH=CH,
R⁵ et R⁶ signifient, indépendamment l'un de l'autre, à chaque fois hydrogène ou méthyle, ou
R⁵ et R⁶ forment ensemble avec l'atome de carbone auquel ils sont liés, le groupe C=O.

2. Composés selon la revendication 1, où
X signifie chlore, brome, fluor ou le groupe méthylsulfonyle,
R¹ et R² signifient, indépendamment l'un de l'autre, à chaque fois hydrogène, (C₁-C₆)-alkyle, halogéno-(C₁-C₆)-alkyle, halogéno-(C₁-C₆)-alcényle, (C₃-C₆)-cycloalkyl-(C₁-C₆)-alkyle ou (C₁-C₆)-alcoxy-(C₁-C₆)-alkyle,
R⁴ et R⁷ signifient, indépendamment l'un de l'autre, à chaque fois hydrogène ou méthyle ;
R³ et R⁸ signifient, indépendamment l'un de l'autre, à chaque fois hydrogène ou méthyle, ou
R³ et R⁸ forment ensemble le groupe CH₂CH₂,
R⁵ et R⁶ signifient, indépendamment l'un de l'autre, à chaque fois hydrogène ou méthyle, ou
R⁵ et R⁶ forment ensemble avec l'atome de carbone auquel ils sont liés, le groupe C=O.

3. Composés selon la revendication 1 ou 2, où
X signifie chlore ou brome,
R¹ signifie hydrogène ou méthyle,
R² signifie hydrogène, (C₁-C₄)-alkyle, halogéno-(C₁-C₄)-alkyle, (C₃-C₆)-cycloalkyl-(C₁-C₄)-alkyle ou (C₁-C₄)-alcoxy-(C₁-C₄)-alkyle,
R⁴ et R⁷ signifient, indépendamment l'un de l'autre, à chaque fois hydrogène ou méthyle ;
R³ et R⁸ signifient, indépendamment l'un de l'autre, à chaque fois hydrogène ou méthyle, ou
R³ et R⁸ forment ensemble le groupe CH₂CH₂,
R⁵ et R⁶ signifient, indépendamment l'un de l'autre, à chaque fois hydrogène ou méthyle, ou
R⁵ et R⁶ forment ensemble avec l'atome de carbone auquel ils sont liés, le groupe C=O.

4. Agents herbicides, **caractérisés par** une teneur active en tant qu'herbicide en au moins un composé de formule (I) selon l'une quelconque des revendications 1 à 3.

5. Agents herbicides selon la revendication 4 en mélange avec des adjuvants de formulation.

6. Agents herbicides selon la revendication 4 ou 5 contenant au moins une autre substance active en tant que pesticide du groupe des insecticides, des acaricides, des herbicides, des fongicides, des antidotes et des régulateurs de croissance.

7. Procédé pour lutter des plantes non souhaitées de plantes, **caractérisé en ce qu'**on applique une quantité active d'au moins un composé de formule (I) selon l'une quelconque des revendications 1 à 3 ou d'un agent herbicide selon l'une quelconque des revendications 4 à 6 sur les plantes ou sur le lieu de la croissance non souhaitée des plantes.

8. Utilisation de composés de formule (I) selon l'une quelconque des revendications 1 à 3 ou d'agents herbicides selon l'une quelconque des revendications 4 à 6 pour lutter contre des plantes non souhaitées.

9. Utilisation selon la revendication 8, **caractérisée en ce que** les composés de formule (I) sont utilisés pour lutter contre des plantes non souhaitées dans les cultures de plantes utiles.

10. Utilisation selon la revendication 9, **caractérisée en ce que** les plantes utiles sont des plantes utiles transgéniques.
